(19) Europäisches Patentamt
European Patent Office
Office européen des brevets

(11) Veröffentlichungsnummer: **0 447 947 A1**

# (12) EUROPÄISCHE PATENTANMELDUNG

(21) Anmeldenummer: **91103883.4**

(22) Anmeldetag: **14.03.91**

(51) Int. Cl.[5]: **C07D 295/096**, A01N 33/08, A01N 33/10, C07C 47/277, C07C 47/27, C07C 43/23, C07C 43/178

(30) Priorität: **23.03.90 DE 4009411**

(43) Veröffentlichungstag der Anmeldung:
**25.09.91 Patentblatt 91/39**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Zipplies, Matthias, Dr.**
**Kastanienweg 1**
**W-6945 Hischberg(DE)**
Erfinder: **Siegel, Hardo, Dr.**
**Hans-Purmann-Allee 25**
**W-6720 Speyer(DE)**
Erfinder: **Becker, Rainer, Dr.**
**Im Haseneck 22**
**W-6702 Bad Duerkheim(DE)**
Erfinder: **Hermeling, Dieter, Dr.**
**Zum Ordenswald 73 f**
**W-6730 Neustadt(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

## (54) N-(3-Phenyl-2-methylpropyl und -methyl-prop-2-enyl)-azaheterocyclen.

(57) N-(3-Phenyl-2-methylpropyl und -methylprop-2-enyl)-azaheterocyclen I

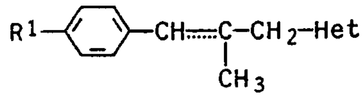

I

(R1 = $(CH_3)_3$-O-, $R^2$-O-$C(CH_3)_2$-, $R^2$ = H, $C_1$-$C_4$-Alkyl; Het = 5-7-gliedriger azahetero-aliphatischer Ring, dessen N-Atom mit dem restlichen Molekülteil von I verbunden ist, wobei der Ring noch ein dem N nicht benachbartes O oder S enthalten und/oder 1-2 der folgenden Substituenten tragen kann: $C_1$-$C_4$-Alkyl, $R^3$-O-$(CH_2)_n$-, $R^3$ = H, $C_1$-$C_4$-Alkyl, $C_1$-$C_2$-Alkoxycarbonyl, n = 0-2, ausgenommen $R^3$-O- in $\alpha$-Position zum N) sowie die N-Oxide und die Salze von I.

Die Verbindungen I eignen sich als Fungizide.

EP 0 447 947 A1

Die vorliegende Erfindung betrifft neue N-(3-Phenyl-2-methylpropyl und -methylprop-2-enyl)- azaheterocyclen der Formel I

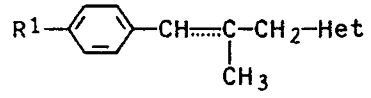

I

in der die punktierte Bindung für eine mögliche zusätzliche Bindung steht und in der die Substituenten folgende Bedeutung haben:

$R^1$ eine tert.-Butoxygruppe oder einen Rest

$R^2$-O-C($CH_3$)$_2$-

wobei $R^2$ Wasserstoff oder $C_1$-$C_4$-Alkyl bedeutet;

Het ein 5- bis 7-gliedriger azahetero-aliphatischer Ring, dessen N-Atom mit dem restlichen Molekülteil von I verbunden ist, wobei der Ring noch ein dem Stickstoff nicht benachbartes Sauerstoff- oder Schwefelatom als weiteres Heteroatom enthalten und/oder noch 1 bis 2 der folgenden Substituenten tragen kann: $C_1$-$C_4$-Alkylgruppen oder Reste $R^3$-O-($CH_2$)n-, wobei $R^3$ Wasserstoff, $C_1$-$C_4$-Alkyl oder $C_1$-$C_2$-Alkoxycarbonyl und n 0, 1 oder 2 bedeutet, mit der Maßgabe, daß der Rest $R^3$-O- sich nicht in α-Position zum Stickstoff des Heterocyclus befindet,

sowie die N-Oxide und die pflanzenverträglichen Säureadditionssalze und quaternären Salze von I.

Außerdem betrifft die Erfindung Verfahren zur Herstellung dieser Verbindungen, ihre Verwendung als Fungizide und fungizide Mittel, welche diese Verbindungen als wirksame Substanzen enthalten.

Des weiteren betrifft die Erfindung 3-Phenyl-2-methylpropan- und 3-Phenyl-2-methylprop-2-en-aldehyde der Formel II'

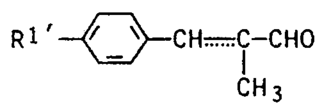

II',

in der $R^{1'}$ eine tert.-Butoxygruppe oder einen Rest HO-C($CH_3$)$_2$- bedeutet, und 3-Phenyl-2-methylpropanole der Formel III

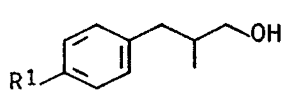

III

als Zwischenprodukte.

Aus dem Schrifttum sind verschiedene fungizid wirksame Verbindungen vom Typ 1 bekannt, die anstelle des Restes $R^1$ eine tert.-Butylgruppe (DE-A-26 56 747, DE-A-27 52 135, EP-A-0 333 und EP-A 243 940), eine tert.-Butylgruppe, die durch Chlor oder Alkyl substituiert sein kann (EP-A-174 565), eine Isopropylgruppe (EP-A-6 992), eine unsubstituierte oder substituierte Phenoxygruppe (EP-A-262 870) oder eine $C_4$-$C_{12}$-Alkylgruppe, die Phenylgruppe, eine Cycloalkyl-, Cycloalkylalkyl- oder Arylalkylgruppe (DE-A 27 52 096) enthalten.

Die vorstehend genannten Verbindungen können jedoch sowohl bezüglich einer guten Pflanzenverträglichkeit als auch bezüglich ihrer fungiziden Wirkung und der Wirkungsdauer - besonders bei kleinen Aufwandmengen und -konzentrationen - nur bedingt befriedigen.

Der Erfindung lag daher die Aufgabe zugrunde, neue fungizid wirksame Substanzen zu finden.

Demgemäß wurden die eingangs definierten N-(3-Phenylisobutyl)-azaheterocyclender Formel I gefunden.

Im einzelnen haben die Substituenten in den erfindungsgemäßen Verbindungen I die folgende Bedeutung:

$R^1$ die tert.-Butoxygruppe oder einen Rest $R^2$-O-C($CH_3$)$_2$ mit

$R^2$ Wasserstoff oder $C_1$-$C_4$-Alkyl wie Methyl, Ethyl, Isopropyl und tert.-Butyl, bevorzugt die tert.-Butoxy- oder die 1-Methoxy-1-methylethylgruppe;

Het einen 5- bis 7-gliedrigen azahetero-aliphatischen Ring, dessen N-Atom mit dem restlichen Molekülteil von I verbunden ist, wobei der Ring noch ein dem Stickstoffatom nicht benachbartes Sauerstoff- oder Schwefelatom als Heteroatom enthalten und/oder 1-2 der folgenden Substituenten tragen kann:

- verzweigtes oder unverzweigtes $C_1$-$C_4$-Alkyl wie Methyl, Ethyl, Isopropyl und tert.-Butyl, bevorzugt Methyl und Ethyl;
- Reste $R^3$-O-$CH_2$- und $R^3$-O-$CH_2$-$CH_2$- oder einen Rest $R^3$-O-, der sich nicht in $\alpha$-Position zum Stickstoffatom des Heterocyclus befindet, mit
  $R^3$
  - Wasserstoff;
  - $C_1$-$C_4$-Alkyl wie Methyl, Ethyl, Isopropyl und tert.-Butyl, bevorzugt Methyl;
  - Methoxycarbonyl und Ethoxycarbonyl;

  insbesondere Pyrrolidin-1-yl, Piperidin-1-yl, Azepan-1-yl, Morpholin-4-yl, Thiomorpholin-4-yl, 2-Methylpyrrolidin-1-yl, 3-Methylpiperidin-1-yl, 4-tert.-Butylpiperidin-1-yl, 3,4-Dimethylpiperidin-1-yl, 3,5-Dimethylpiperidin-1-yl, 2,6-Dimethylpiperidin-1-yl, 3-Methyl-4-ethylpiperidin-1-yl, 3-Ethyl-4-methylpiperidin-1-yl, 2-Methylazepan-1-yl, 3-Methylazepan-1-yl, 2,5-Dimethylmorpholin-4-yl, cis-2,6-Dimethylmorpholin-4-yl, 2,6-Dimethylmorpholin-4-yl (cis-/trans-Gemisch), 3-Hydroxypiperidin-1-yl, 4-Hydroxypiperidin-1-yl, 4-Methoxypiperidin-1-yl, 3-Hydroxymethylpiperidin-1-yl, 4-Hydroxymethylpiperidin-1-yl, 3-Methoxymethylpiperidin-1-yl, 4-Methoxymethylpiperidin-1-yl, 3-Ethoxymethylpiperidin-1-yl, 3-Propoxymethylpiperidin-1-yl, 3-Acetoxymethylpiperidin-1-yl, 3-Propionoxymethylpiperidin-1-yl, 4-Methoxyethylpiperidin-1-yl und 3-Acetoxyethylpiperidin-1-yl.

Besonders gut geeignet sind Verbindungen I, bei denen $R^1$ die tert.-Butoxy- oder die 1-Methoxy-1-methylethylgruppe und Het den Piperidin-1-yl-, Azepan-1-yl-, Morpholin-4-yl, 2,6-Dimethylpiperidin-1-yl- oder cis-2,6-Dimethylmorpholin-4-yl-Rest bedeuten.

Bevorzugte Verbindungen I sind den Beispielen zu entnehmen.

Die N-(3-Phenyl-2-methylpropyl und -methylprop-2-enyl)-azaheterocyclen sind auf verschiedene Weise erhältlich, und zwar vorzugsweise nach folgenden Methoden:

1) Reduktion eines Aldehyds II in Gegenwart eines azaheterocyclischen Ringes H-Het

1a) Katalytische Reduktion mit Wasserstoff

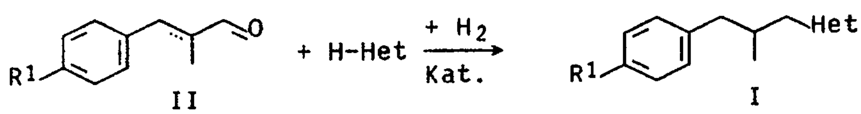


II I

Die Hydrierung wird zweckmäßig in einem Autoklaven bei einem Wasserstoffdruck von 2 bis 100 bar, bevorzugt 90 bis 100 bar, durchgeführt. In der Regel arbeitet man lösungsmittelfrei oder in einem inerten Löungs- bzw. Verdünnungsmittel wie Benzol, Toluol, Tetrahydrofuran, Dioxan und Methanol bei einer Reaktionstemperatur zwischen 0 und 180 °C, bevorzugt 30 bis 110 °C.

Zweckmäßigerweise setzt man den Aldehyd II und den Azaheterocyclus H-Het im stöchiometrischen Verhältnis ein, jedoch kann in manchen Fällen auch ein Überschuß der einen oder anderen Komponente, etwa bis zu 10 %, empfehlenswert sein.

Je nach verwendetem Katalysatortyp können Verbindungen I mit einer 2-Methylpropylen- oder einer 2-Methylprop-2-enylengruppe als Brückenglied erhalten werden. Bevorzugt werden Katalysatoren, die zu Verbindungen I mit einem gesättigten Brückenglied führen.

Als Katalysatoren für die Hydrierung gut geeignet sind Edelmetalle wie Rhodium und vor allem Palladium, und zwar vorzugsweise in Form von Trägerkatalysatoren mit Silikaten wie Aluminium- und Magnesium-silikate, sowie besonders Aluminiumoxid, Titandioxid oder Kohle als Trägermaterialien.

In der Regel beträgt die Katalysatormenge 1 bis 50 %, bevorzugt 2 bis 10 %, bezogen auf die Menge an eingesetztem Aldehyd II.

Ein besonders gut geeignetes Verfahren zur Herstellung der Verbindungen I mit einer 2-Methylpropylengruppe als Brückenglied besteht in der Umsetzung eines Aldehydes $R^1$-Phenyl-4-CHO mit Propionaldehyd und einem Azaheterocyclus H-Het unter hydrierenden Bedingungen, wie es in der

DE-A-31 05 446 beschrieben ist.

1b) Katalytische Reduktion mit einem Metallhydrid

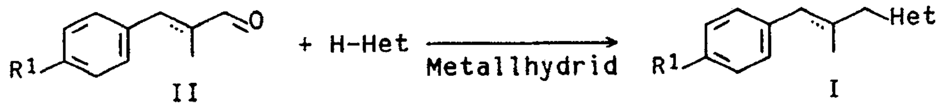


Die Reduktion erfolgt zweckmäßigerweise in einem inerten Lösungs- bzw. Verdünnungsmittel, beispielsweise einem Alkohol wie Methanol und Ethanol.

Als Metallhydride eignen sich z.B. Alkali- oder Erdalkalimetallhydride oder die Alkalimetallsalze von Borhydriden oder Aluminiumhydriden. Besonders gut geeignet ist Natriumcyanborhydrid, dem noch bis zu 0,5 Moläquivalente eines Co-Katalysators wie Zink(II)-chlorid zugesetzt werden können.

In der Regel ist eine Menge von 2 bis 4 Äquivalenten an Reduktionsmittel, bezogen auf die Menge an eingesetztem Aldehyd II, ausreichend.

Aldehyd II und Azaheterocyclus H-Het setzt man normalerweise in stöchiometrischem Verhältnis ein, jedoch kann in manchen Fällen auch ein Überschuß der einen oder anderen Komponente, etwa bis zu 10 %, empfehlenswert sein.

Normalerweise arbeitet man bei Atmosphärendruck oder unter dem Eigendruck des jeweiligen Lösungsmittels.

Die Reaktionstemperatur liegt im allgemeinen zwischen 0 und 80 °C.

1c) Reduktion mit organischen Reduktionsmitteln

Als organisches Reduktionsmittel eignet sich insbesondere Ameisensäure.

Bezüglich der Lösungsmittel, der stöchiometrischen Mengen und des Druckes gelten die Angabe für Methode 1b).

Im Falle flüssiger Reduktionsmittel kann die Reaktion auch ohne Lösungsmittel in einem großen Überschuß des Reduktionsmittels, etwa bis zu 10 %, bezogen auf die Menge an Aldehyd II, durchgeführt werden.

Die Reaktionstemperatur liegt im allgemeinen zwischen 80 und 180 °C, bevorzugt zwischen 120 und 160 °C.

Normalerweise arbeitet man bei Normaldruck.

2) Nucleophile Substitution durch einen Azaheterocyclus

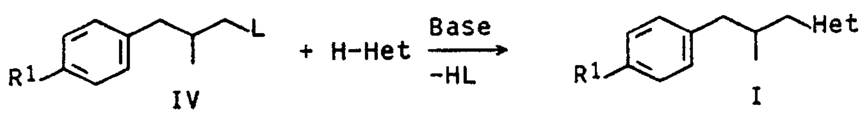


L bedeutet eine nucleophil substituierbare Abgangsgruppe, insbesondere ein Halogenatom wie Chlor, Brom und Jod, einen Sulfonyl-Rest wie Methansulfonyl, p-Toluolsulfonyl, p-Bromsulfonyl oder einen Methylsulfat-Rest.

Zweckmäßigerweise nimmt man die Umsetzung in einem Lösungs- bzw. Verdünnungsmittel wie Methanol, Ethanol, Acetonitril, Dimethylformamid, Dimethylsulfoxid oder auch in einem Überschuß des Azaheterocyclus vor.

Als Basen eignen sich beispielsweise Alkalimetallcarbonate wie Natrium- und Kaliumcarbonat, Hydrogencarbonate wie Natrium- und Kaliumhydrogencarbonat, Pyridin, 4-Dimethylaminopyridin und Triethylamin oder auch H-Het selber, und zwar für eine vollständige Umsetzung in mindestens stöchiometrischer Menge, bezogen auf die Menge an III.

Vorteilhaft setzt man alle Ausgangsverbindungen in etwa stöchiometrischem Verhältnis ein, jedoch kann in manchen Fällen auch ein Überschuß der einen oder anderen Komponente, etwa bis zu 10 %, empfehlenswert sein.

Verwendet man den Azaheterocyclus als Lösungsmittel und/oder als Base, so liegt H-Het in einem größeren Überschuß vor.

Die Reaktion verläuft normalerweise oberhalb von 30 °C mit ausreichender Geschwindigkeit. Im allgemeinen liegt die Temperatur zwischen 40 und 160 °C, bevorzugt zwischen 50 und 130 °C.

Da die Reaktion nicht druckabhängig ist, arbeitet man vorteilhaft bei Normaldruck.

3) Veretherung von Hydroxyverbindungen I [$R^1$ = $R^2$-O-C($CH_3$)$_2$-]

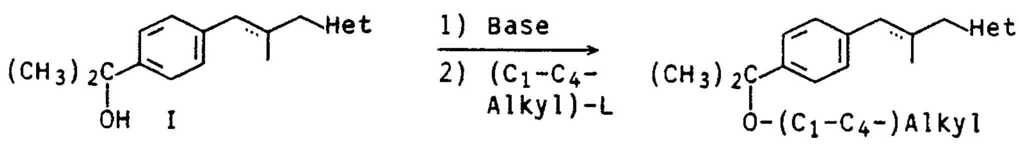


I I

Die Umsetzung erfolgt zweckmäßig in einem inerten Lösungsmittel wie Dimethylformamid.

In der Regel überführt man den Alkohol durch Umsetzung mit einer starken Base, beispielsweise Natriumhydrid oder Methylmagnesiumbromid, bei etwa 0 bis 40 °C in sein Alkoholat.

Die anschließende Substitution der Abgangsgruppe L durch das Alkoholat-Ion verläuft normalerweise oberhalb 0 °C mit ausreichender Geschwindigkeit.

Im allgemeinen liegt die Temperatur zwischen 20 und 100 °C.

Vorteilhaft setzt man die Ausgangsverbindungen im stöchiometrischen Verhältnis ein, jedoch kann in manchen Fällen auch ein Überschuß der einen oder anderen Komponente, etwa bis zu 10% empfehlenswert sein.

Da die Reaktion nicht druckabhängig ist, arbeitet man bevorzugt bei Normaldruck.

Beispiele für die Herstellung von Verbindungen vom Typ I, die am Azaheterocyclus eine Hydroxy-, Hydroxyalkyl- oder Alkoxyalkylgruppe tragen, können der EP-A 174 565 entnommen werden.

Die Ausgangsverbindungen II sind nach an sich bekannten Verfahren der Aldolkondensationsreaktion aus para-substituierten aromatischen Aldehyden mit Propanaldehyd und gewünschtenfalls anschließender partieller Hydrierung erhältlich:

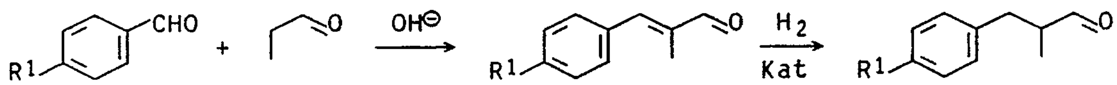


II II

Die gesättigten Aldehyde II können ebenfalls nach an sich bekannten Verfahren zu Alkoholen III reduziert werden, aus denen sich durch Umsetzung mit einer Mineralsäure bzw. einer reaktiven Verbindung HL auf an sich bekannte Weise die Zwischenprodukte IV herstellen lassen:

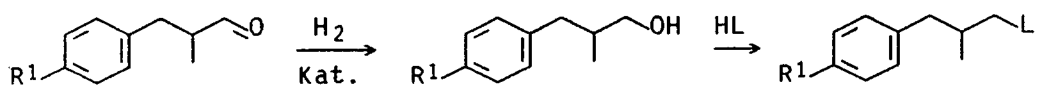


II (gesättigt) III IV

Unter den Aldehyden II sind 3-[4-(1-Hydroxy-1-methylethyl)phenyl]-2-methylpropenal, 3-[4-(1-Hydroxy-1-methylethyl)phenyl]-2-methylpropanal, 3-[4-tert.-Butoxyphenyl]-2-methylpropenal und 3-[4-tert.-Butoxyphenyl]-2-methylpropanal neu.

Bei der Herstellung von Verbindungen I mit einer Doppelbindung im Isobutylteil entstehen überwiegend die E-Isomeren und in geringerer Menge auch die Z-Isomeren. Beide Isomeren können gewünschtenfalls wie üblich voneinander getrennt werden.

Die N-Oxide können nach üblichen Methoden aus den Verbindungen I, z.B. durch deren Umsetzung mit einer organischen Persäure wie Metachlorperbenzoesäure, hergestellt werden.

Als Säureadditionssalze eignen sich die Salze von solchen Säuren, welche die fungizide Wirkung von I nicht beeinträchtigen, also z.B. die Salze der Chlorwasserstoffsäure, Bromwasserstoffsäure, Schwefelsäure, Salpetersäure, Phosphorsäure, Borsäure, Ameisensäure, Essigsäure, Propionsäure, Laurylsäure, Palmitinsäure, Stearinsäure, Oxalsäure, Äpfelsäure, Malonsäure, Benzoesäure, Methansulfonsäure, Dodecylbenzolsulfonsäure sowie der Saccarinsäure. Weitere geeignete Salze sind die N-alkylierten, insbesondere methylierten und ethylierten, sowie die N-benzylierten Derivate der Verbindungen I.

Die erfindiungsgemäßen Verbindungen I mit gesättigtem Brückenglied enthalten ein Asymmetriezentrum. Außerdem können alle Säureadditionssalze, quartären Salze und N-Oxide 1 Asymmetriezentrum am Stickstoffatom des Heterocyclus enthalten. Alle opt. akt. Verbindungen wirken als Racemate, wie sie bei den meisten Herstellungsverfahren anfallen, können gewünschtenfalls aber auch nach den hierfür üblichen

Methoden, z.B. durch Chromatographie an einem optisch aktiven Adsorptionsmittel, in die reinen Isomeren aufgetrennt werden.

Die Verbindungen I eignen sich als Fungizide.

Herstellungsbeispiele

Beispiel 3

4-[3-(4-tert.-Butoxyphenyl)-2-methylpropyl]-cis-2,6-dimethylmorpholin

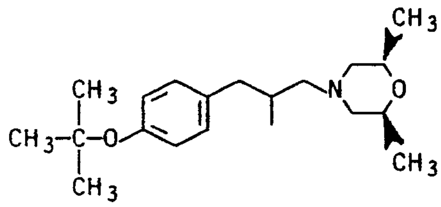

Eine Mischung aus 25 g (0,1 mol) [3-(4-tert.-Butoxyphenyl)-2-methylpropyl]-p-toluolsulfonat, 25 g (0,22 mol) cis-2,6-Dimethylmorpholin und 100 ml Toluol wurde 4 Stunden lang auf Rückflußtemperatur erhitzt und danach mit 10 gew.-%iger wäßriger Kalilauge gewaschen. Die organische Phase wurde anschließend wie üblich auf das Produkt hin aufgearbeitet. Ausbeute: 60 % d.Th.; Sdp. 120 °C/0,1 mbar.

Vorstufe A

3- (4-tert.-Butoxyphenyl)-2-methylpropan-1-ol

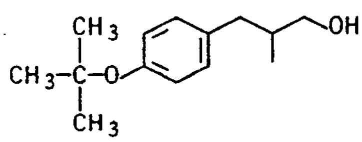

Zu einer Lösung aus 600 ml Methanol und 13,3 g 50 %iger wäßriger Natronlauge wurden im Verlauf von ca. 10 min 10 g (0,58 mol) p-tert.-Butoxybenzaldehyd gegeben, wobei man einen schwachen Stickstoffstrom durch die Lösung leitete. Nach Erhitzen der Mischung auf ca. 40 °C wurden dann im Verlauf von 1 Stunde 36,4 g (0,62 mol) Propionaldehyd zugetropft. Nachdem man noch 30 min gerührt hatte wurde mit 10 %iger wäßriger Schwefelsäure ein pH-Wert zwischen 7,5 und 8 eingestellt und gebildetes Natriumsulfat abfiltriert. Das entstandene 3-(4-tert.-Butoxyphenyl)-2-methylpropenal wurde unter Verwendung von 15 g (0,26 mol) Raney-Nickel als Katalysator 3 Stunden bei einem Wasserstoffdruck von 100 bar und einer Temperatur von 60 °C hydriert. Nach Entspannung und Abkühlung wurde der Inhalt des Autoklaven wie üblich auf das Produkt hin aufgearbeitet. Ausbeute: 66 %; Sdp.: 133 °C/0,5 mbar.

Vorstufe B

[3-(4-tert.-Butoxyphenyl)-2-methylpropyl]-p-toluolsulfonat

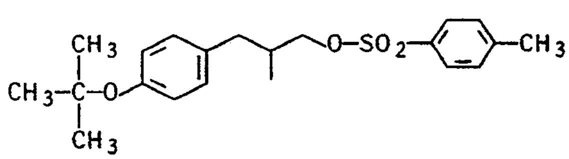

Zu einer Mischung aus 77 g (0,34 mol) 3-(4-tert.-Butoxyphenyl)-2-methylpropan-1-ol in 150 ml Pyridin wurden bei etwa 20 °C 66 g (0,34 mol) p-Toluolsulfonsäurechlorid zugegeben. Nach 2-stündigem Rühren gab man ca. 250 ml Toluol und 250 ml Wasser zu, trennte die organische Phase ab und entfernte das Lösungsmittel bei reduziertem Druck. Das Rohprodukt wurde ohne weitere Reinigung mit dem Azaheterocyclus zum Endprodukt umgesetzt.

Beispiel 8

4-(3-[4-(1-Methoxy-1-methylethyl)phenyl]-2-methylprop-2-en-1-yl]-cis-2,6--dimethylmorpholin

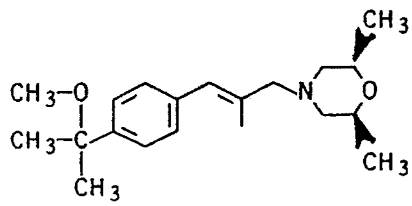

Eine Mischung aus 10,0 g (46 mmol) 3-[4(1-Methoxy-1-methylethyl)phenyl]-2-methylpropenaldehyd [hergestellt analog Beispiel 3, Vorstufe A ohne die Hydrierung (Ausbeute 87 % d.Th., Sdp. 120 °C/0,2 mbar)], 5,6 g (48,6 mmol) cis-2,6-Dimethylmorpholin, 3,7 g (27 mmol) Zinkchlorid, 3,4 g (54 mmol) Natriumcyanborhydrid und 100 ml abs. Methanol wurde unter Stickstoffschutz 48 Stunden bei etwa 20 °C gerührt. Anschließend wurde das Lösungsmittel bei reduziertem Druck entfernt. Den Rückstand nahm man in ca. 150 ml Methyl-tert.-butylether und 40 ml verdünnter Natronlauge auf, trennte die Phasen und arbeitete den organischen Anteil wie üblich auf das Produkt hin auf. Ausbeute: 54 % d.Th. ; Sdp. 150 °C/0, 3 mbar.

Beispiel 9

4-(3-[4-(1-Methoxy-1-methylethyl)phenyl]-2-methylpropyl]-cis-2,6-dimethylmorpholin

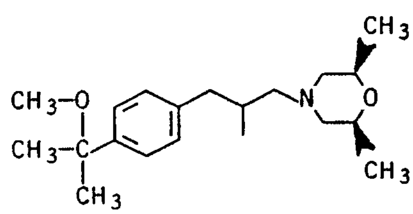

Die Herstellung der Verbindung erfolgte analog dem Beispiel 8 aus 10 g (45 mmol) 3-[4-(1-Methoxy-1-methylethyl)-phenyl]-2-methylpropanaldehyd und 6,7 g (58 mmol) cis-2,6-Dimethylmorpholin, unter Verwendung von 3,7 g (27 mmol) Zinkchlorid und 3,4 g (54 mmol) Natriumcyanoborhydrid. Ausbeute: 76 % d.Th. ; Sdp. 145 °C/0,4 mbar.

Vorstufe C

3-[4-(1-Methoxy-1-methylethyl)phenyl]-2-methylpropanaldehyd

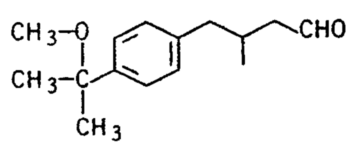

100 g (0,46 mol) 3-[4-(1-Methoxy-1-methylethyl)phenyl]-2-methylpropenal [hergestellt analog Beispiel 3,

Vorstufe A ohne Durchführung der Hydrierung (Sdp. 120 °C/0,2 mbar)] in 50 g Methanol wurden 12 Stunden lang bei 80 °C und einem Wasserstoffdruck von 50 bar an einem Metallkontakt hydriert. Dieser bestand aus Palladium (0,5 %) auf Aluminiumoxid. Nach Abkühlung und Entspannung wurde wie üblich auf das Produkt hin aufgearbeitet. Ausbeute: 62 % d.Th.; Sdp. 90 bis 94 °C/0, 2 mbar.

In Tabelle 1 sind noch weitere Verbindungen I enthalten, die analog den Beispielverbindungen hergestellt wurden oder erhältlich sind.

Tabelle 1

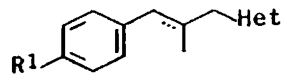

(I)

| Nr. | $R^1$ | Doppelbindung | Het | Sdp. [°C/mbar] |
|---|---|---|---|---|
| 1 | tert.-Butoxy | nein | Pyrrolidinyl | |
| 2 | tert.-Butoxy | nein | 4-tert.-Butylpiperidinyl | |
| 3 | tert.-Butoxy | nein | cis-2,6-Dimethylmorpholinyl | 120/0,1 |
| 4 | tert.-Butoxy | nein | Piperidinyl | 140/0,4 |
| 5 | tert.-Butoxy | nein | Azepanyl | 113/0,1 |
| 6 | $CH_3O-C(CH_3)_2-$ | nein | Pyrrolidinyl | |
| 7 | $CH_3O-C(CH_3)_2-$ | nein | 4-tert.-Butylpiperidinyl | |
| 8 | $CH_3O-C(CH_3)_2-$ | ja | cis-2,6-Dimethylmorpholinyl | 150/0,3 |
| 9 | $CH_3O-C(CH_3)_2-$ | nein | cis-2,6-Dimethylmorpholinyl | 145/0,4 |
| 10 | $CH_3O-C(CH_3)_2-$ | nein | Piperidinyl | 130/0,4 |
| 11 | $CH_3O-C(CH_3)_2-$ | ja | Piperidinyl | 150/0,3 |
| 12 | $CH_3O-C(CH_3)_2-$ | nein | Azepanyl | 140/0,4 |
| 13 | $CH_3O-C(CH_3)_2-$ | ja | Azepanyl | 170/0,4 |
| 14 | $CH_3O-C(CH_3)_2-$ | nein | Morpholinyl | |
| 15 | tert.-Butoxy | nein | Morpholinyl | |
| 16 | tert.-Butoxy | nein | 3-Methyl-piperidinyl | |
| 17 | tert.-Butoxy | nein | 3-Hydroxymethyl-piperidinyl | |
| 18 | tert.-Butoxy | nein | 3-Methoxymethylpiperidinyl | |
| 19 | tert.-Butoxy | nein | 3-Ethoxymethylpiperidinyl | |
| 20 | tert.-Butoxy | nein | 3-Acetoxymethyl-piperidinyl | |
| 21 | tert.-Butoxy | nein | 4-Hydroxypiperidinyl | |
| 22 | tert.-Butoxy | nein | 4-Methoxypiperidinyl | |

Tabelle 1 - Forts.

| Nr. | R1 | Doppelbindung | Het | Sdp. [°C/mbar] |
|---|---|---|---|---|
| 23 | tert.-Butoxy | nein | 4-Ethoxypiperidinyl | |
| 24 | tert.-Butoxy | nein | 4-Acetoxypiperidinyl | |
| 25 | tert.-Butoxy | nein | 4-Propionoxypiperidinyl | |
| 26 | tert.-Butoxy | nein | 3-Methoxyethyl-piperidinyl | |
| 27 | $CH_3O-C(CH_3)_2-$ | nein | 3-Methyl-piperidinyl | |
| 28 | $CH_3O-C(CH_3)_2-$ | nein | 3,5-Dimethylpiperidinyl | |
| 29 | $CH_3O-C(CH_3)_2-$ | nein | 3-Hydroxymethyl-piperidinyl | |
| 30 | $CH_3O-C(CH_3)_2-$ | nein | 3-Methoxymethylpiperidinyl | |
| 31 | $CH_3O-C(CH_3)_2-$ | nein | 3-Ethoxymethylpiperidinyl | |
| 32 | $CH_3O-C(CH_3)_2-$ | nein | 3-Acetoxymethylpiperidinyl | |
| 33 | $CH_3O-C(CH_3)_2-$ | nein | 4-Hydroxypiperidinyl | |
| 34 | $CH_3O-C(CH_3)_2-$ | nein | 4-Hydroxymethylpiperidinyl | |
| 35 | $CH_3O-C(CH_3)_2-$ | nein | 4-Methoxypiperidinyl | |
| 36 | $CH_3O-C(CH_3)_2-$ | nein | 4-Ethoxypiperidinyl | |
| 37 | $CH_3O-C(CH_3)_2-$ | nein | 4-Acetoxypiperidinyl | |
| 38 | $C_2H_5O-C(CH_3)_2-$ | nein | 2,6-Dimethylmorpholinyl | |
| 39 | $C_2H_5O-C(CH_3)_2-$ | nein | Piperidinyl | |
| 40 | $C_2H_5O-C(CH_3)_2-$ | nein | Hexamethyleniminyl | |

Die N-(3-Phenyl-2-methylpropyl und -methylprop-2-enyl)-azaheterocyclen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen

Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Ganz besonders gut geeignet sind die Verbindungen I zur Bekämpfung von Pyricularia oryzae an Reis.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des N-(3-Phenyl-2-methylpropyl und -methylprop-2-enyl)-azaheterocycluses gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. eine Lösung aus 90 Gew.-Teilen der Verbindung Nr. 3 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 4, 80 Gew.-Teilen xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion;

III. eine wäßrige Dispersion aus 20 Gew.-Teilen der Verbindung Nr. 5, 40 Gew.-Teilen Cyclohexanon, 30

Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 8, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 9, 3 Gew.-Teilen des Natriumsalzes der Diisobutylnaphtalin-α-sulfonsäure, 10 Gew.-Teilen des Natrium-salzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 10 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 11, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 12, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 13, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln. Beim Vermischen mit Fungiziden erhält man dabei in vielen Fällen eine Vergrößerung des fungiziden Wirkungsspektrums.

Anwendungsbeispiele

Als Vergleichssubstanz diente

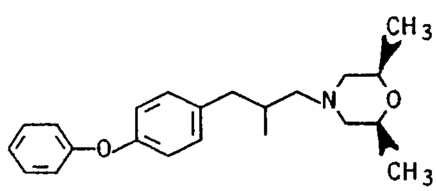

bekannt aus der EP-A 262 870 (Verbindung Nr. 7).

Beispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit 90 bis 95 % relativer Luftfeuchtigkeit gestellt. Während dieser Zeit keimten die Sporen aus und die Keimschläuche drangen in das Blattgewebe ein. Die infizierten Pflanzen wurden mit 0,025 %iger wäßriger Spritzbrühe, die 80 % Wirkstoff und 20 % Emulgiermittel in der Trockensubstanz enthielten, tropfnaß gespritzt und nach dem Antrocknen des Spritzbelages im Gewächshaus bei einer Temperatur zwischen 20 und 22°C und 65 bis 70 % relativer Luftfeuchtigkeit aufgestellt. Nach 8 Tagen beurteilte man das Ausmaß der Rostpilzentwicklung auf den Blättern.

Das Ergebnis zeigt, daß die Wirkstoffe 9, 10, 11 und 13 bei der Anwendung als 0,025 %ige (Gew.-%) Spritzbrühe eine bessere fungizide Wirkung aufweisen (95 %) als die bekannte Vergleichssubstanz (30 %).

Beispiel 2

Wirksamkeit gegen Weizenmehltau

Blätter von in Töpfen gewachsenen Weizenkeimlingen der Sorte "Frühgold" wurden mit 0,025 %iger wäßriger Spritzbrühe, die 80 % Wirkstoff und 20 % Emulgiermittel in der Trockenmasse enthielten, besprüht und 24 Stunden nach dem Antrocknen des Spritzbelages mit Sporen des Weizenmehltaus (Erysiphe graminis var. tritici) bestäubt. Die Versuchspflanzen wurden anschließend im Gewächshaus bei Temperaturen zwischen 20 und 22° C und 75 bis 80 % relativer Luftfeuchtigkeit aufgestellt. Nach 7 Tagen wurde das Ausmaß der Mehltauentwicklung beurteilt.

Das Ergebnis zeigt, daß die Wirkstoffe 3, 4, 5, 9, 10, 11 und 13 bei der Anwendung als 0,025 %ige (Gew.-%) Spritzbrühe eine bessere fungizide Wirkung aufweisen (98 %) als die bekannte Vergleichssubstanz (50 %).

**Patentansprüche**

1. N-(3-Phenyl-2-methylpropyl und -methylprop-2-enyl)-azaheterocyclen der allgemeinen Formel I

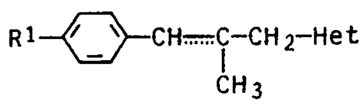

I

in der die punktierte Bindung für eine mögliche zusätzliche Bindung steht und in der die Substituenten folgende Bedeutung haben:

$R^1$ eine tert.-Butoxygruppe oder einen Rest

$R^2-O-C(CH_3)_2-$

wobei $R^2$ Wasserstoff oder $C_1$-$C_4$-Alkyl bedeutet;

Het ein 5- bis 7-gliedriger azahetero-aliphatischer Ring, dessen N-Atom mit dem restlichen Molekülteil von 1 verbunden ist, wobei der Ring noch ein dem Stickstoff nicht benachbartes Sauerstoff- oder Schwefelatom als weiteres Heteroatom enthalten und/oder noch 1 bis 2 der folgenden Substituenten tragen kann: $C_1$-$C_4$-Alkylgruppen oder Reste $R^3-O(CH_2)n-$, wobei

$R^3$ Wasserstoff, $C_1$-$C_4$-Alkyl oder $C_1$-$C_2$-Alkoxycarbonyl und n 0, 1 oder 2 bedeutet, mit der Maßgabe, daß der Rest $R^3$-O- sich nicht in $\alpha$-Position zum Stickstoff des Heterocyclus befindet,

sowie die N-Oxide und die pflanzenverträglichen Säureadditionssalze und quaternären Salze von 1.

2. Verfahren zur Herstellung der N-(3-Phenyl-2-methylpropyl und -methylprop-2-enyl)-azaheterocyclen gemäß Anspruch 1, dadurch gekennzeichnet, daß man einen Aldehyd der Formel II

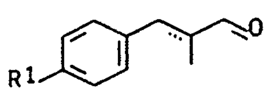

II

in Gegenwart eines azaheterocyclischen Ringes H-Het reduziert und gewünschtenfalls in die N-Oxide oder die pflanzenverträglichen Säureadditionssalze oder quartären Salze überführt.

3. Verfahren zur Herstellung der N-(3-Phenylisobutyl)-azaheterocyclen I gemäß Anspruch 1, dadurch gekennzeichnet, daß man einen 3-Phenyl-2-methylpropanol der Formel III

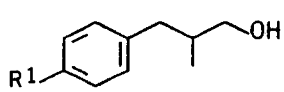

III

in ein 3-Phenyl-2-methylpropanderivat der Formel IV

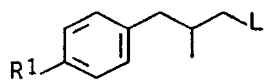

IV

wobei L eine nucleophil substituierbare Abgangsgruppe bedeutet, überführt und diese Verbindung mit einem Azaheterocyclus H-Het in Gegenwart einer Base umsetzt und gewünschtenfalls in die N-Oxide oder die pflanzenverträglichen Säureadditionssalze oder quartären Salze überführt.

**4.** Verwendung der N-(3-Phenyl-2-methylpropyl und -methylprop-2-enyl)-azaheterocyclen I, deren N-Oxiden und pflanzenverträglichen Hydrosalzen und quartären Salzen gemäß Anspruch 1 als Fungizide.

**5.** Fungizides Mittel, enthaltend mindestens einen N-(3-Phenyl-2-methylpropyl und -methylprop-2-enyl)-azaheterocyclus der Formel I, dessen N-Oxid oder dessen pflanzenverträgliches Hydrosalz oder quartäres Salz gemäß Anspruch 1 und einen flüssigen oder festen Trägerstoff.

**6.** Verfahren zur Bekämpfung von Pilzen, dadurch gekennzeichnet, daß man eine fungizid wirksame Menge eines N-(3-Phenyl-2-methylpropyl und -methylprop-2-enyl)-azaheterocyclus I, von dessen N-Oxiden und/oder pflanzenverträglichen Hydrosalzen oder quartären Salzen gemäß Anspruch 1, auf Pilze, von Pilzbefall bedrohte Pflanzen, deren Lebensraum oder auf das Saatgut der bedrohten Pflanzen einwirken läßt.

**7.** N-[3-(4-tert.-Butoxyphenyl)-2-methylpropyl]-piperidin.

**8.** N-[3-(4-(1-Methoxy-2-methylethyl)phenyl)-2-methylpropyl]-piperidin.

**9.** 4-[3-(4-tert.-Butoxyphenyl)-2-methylpropyl]-cis-2,6-dimethylmorpholin.

**10.** 4-(3-(4-(1-Methoxy-2-methylethyl)phenyl)-2-methylpropyl]-cis-2,6-dimethylmorpholin.

**11.** 3-Phenyl-2-methylpropan- und 3-Phenyl-2-methylprop-2-enaldehyde der allgemeinen Formel II'

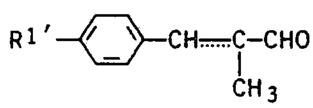

II'

in der $R^{1'}$ eine tert.-Butoxygruppe oder einen Rest $HO-C(CH_3)_2$-bedeutet.

**12.** 3-Phenyl-2-methylpropanole der allgemeinen Formel III

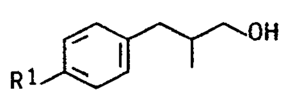

III

in der $R^1$ die in Anspruch 1 genannte Bedeutung hat.

**Patentansprüche für folgenden Vertragsstaat : ES**

**1.** Fungizides Mittel, enthaltend einen flüssigen oder festen Trägerstoff und mindestens einen N-(3-Phenyl-2-methylpropyl und -methylprop-2-enyl)-azaheterocyclus der allgemeinen Formel I

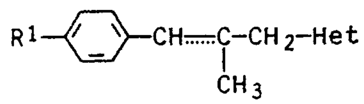

I

in der die punktierte Bindung für eine mögliche zusätzliche Bindung steht und in der die Substituenten folgende Bedeutung haben:

$R^1$ eine tert.-Butoxygruppe oder einen Rest

$R^2$-O-C($CH_3$)$_2$-

wobei $R^2$ Wasserstoff oder $C_1$-$C_4$-Alkyl bedeutet;

Het ein 5- bis 7-gliedriger azahetero-aliphatischer Ring, dessen N-Atom mit dem restlichen Molekülteil von I verbunden ist, wobei der Ring noch ein dem Stickstoff nicht benachbartes Sauerstoff- oder Schwefelatom als weiteres Heteroatom enthalten und/oder noch 1 bis 2 der folgenden Substituenten tragen kann: $C_1$-$c_4$-Alkylgruppen oder Reste $R^3$-O-($CH_2$)n-, wobei

$R^3$ Wasserstoff, $C_1$-$C_4$-Alkyl oder $C_1$-$C_2$-Alkoxycarbonyl und n 0, 1 oder 2 bedeutet, mit der Maßgabe, daß der Rest $R^3$-O- sich nicht in α-Position zum Stickstoff des Heterocyclus befindet,

dessen N-Oxid oder dessen pflanzenverträgliches Säureadditionssalz oder quartäres Salz.

**2.** Fungizides Mittel, enthaltend einen flüssigen oder festen Trägerstoff und N-[3-(4-tert.-Butoxyphenyl)-2-methylpropyl]-piperidin.

**3.** Fungizides Mittel, enthaltend einen flüssigen oder festen Trägerstoff und N-[3-(4-(1-Methoxy-2-methylethyl)-phenyl)-2-methylpropyl]-piperidin.

**4.** Fungizides Mittel, enthaltend einen flüssigen oder festen Trägerstoff und 4-[3-(4-tert.-Butoxyphenyl)-2-methylpropyl]-cis-2,6-dimethylmorpholin.

**5.** Fungizides Mittel, enthaltend einen flüssigen oder festen Trägerstoff und 4-[3-(4-(1-Methoxy-2-methylethyl)-phenyl)-2-methylpropyl]-cis-2,6-dimethylmorpholin.

**6.** Verfahren zur Herstellung der N-(3-Phenyl-2-methylpropyl und -methylprop-2-enyl)-azaheterocyclen gemäß Anspruch 1, dadurch gekennzeichnet, daß man einen Aldehyd der Formel II

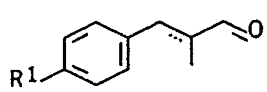

II

in Gegenwart eines azaheterocyclischen Ringes H-Het reduziert und gewünschtenfalls in die N-Oxide oder die pflanzenverträglichen Säureadditionssalze oder quartären Salze überführt.

**7.** Verfahren zur Herstellung der N-(3-phenylisobutyl)-azaheterocyclen I gemäß Anspruch 1, dadurch gekennzeichnet, daß man einen 3-Phenyl-2-methylpropanol der Formel III

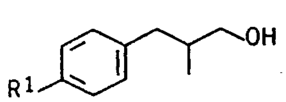

III

in ein 3-Phenyl-2-methylpropanderivat der Formel IV

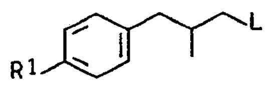

IV

wobei L eine nucleophil substituierbare Abgangsgruppe bedeutet, überführt und diese Verbindung mit einem Azaheterocyclus H-Het in Gegenwart einer Base umsetzt und gewünschtenfalls in die N-Oxide oder die pflanzenverträglichen Säureadditionssalze oder quartären Salze überführt.

8. Verfahren zur Bekämpfung von Pilzen, dadurch gekennzeichnet, daß man eine fungizid wirksame Menge eines N-(3-Phenyl-2-methylpropyl und -methylprop-2-enyl)-azaheterocyclus I, von dessen N-Oxiden und/oder pflanzenverträglichen Hydrosalzen oder quartären Salzen gemäß Anspruch 1, auf Pilze, von Pilzbefall bedrohte Pflanzen, deren Lebensraum oder auf das Saatgut der bedrohten Pflanzen einwirken läßt.

Europäisches Patentamt

# EUROPÄISCHER RECHERCHENBERICHT

Nummer der Anmeldung

EP 91 10 3883

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 058 326 (BASF AG)<br>* Seite 8; Anspruch 1 * | 1,2,11 | C 07 D 295/096<br>A 01 N 33/08<br>A 01 N 33/10<br>C 07 C 47/277<br>C 07 C 47/27<br>C 07 C 43/23<br>C 07 C 43/178 |
| X | EP-A-0 032 659 (BASF AG)<br>* Seite 8; Ansprüche 1-4; Seite 2, Zeile 4 - Seite 3, Zeile 10; Seite 5, Zeilen 14-27 * | 1-12 | |
| X | EP-A-0 007 093 (BASF AG)<br>* Seite 2, Zeile 6 - Seite 3, Zeile 1 * | 1,2,11 | |
| P,X | EP-A-0 392 258 (FIRMENICH SA)<br>* Seite 4, Zeile 54 - Seite 5, Zeile 41; Seite 1, Zeilen 1-26 * | 1-6,8,10, 11 | |

RECHERCHIERTE SACHGEBIETE (Int. Cl.5)

C 07 D 295/00
A 01 N 33/00

Der vorliegende Recherchenbericht wurde für alle Patentansprüche erstellt

| Recherchenort | Abschlußdatum der Recherche | Prüfer |
|---|---|---|
| Den Haag | 17 Juni 91 | PAUWELS G.R.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument